# EUROPEAN PATENT SPECIFICATION

(11) **EP 3 725 220 B1**
(45) Date of publication and mention of the grant of the patent: **25.09.2024**
(21) Application number: 20178793.4
(22) Date of filing: 16.01.2017
(51) Int. Cl.: A61B 5/361, A61B 5/287, G16H 50/20, G16H 30/40

(54) **SYSTEM FOR DETERMINING ATRIAL FIBRILLATION REGIONS FOR ABLATION**
SYSTEM ZUR BESTIMMUNG VON VORHOFFLIMMERREGIONEN ZUR ABLATION
SYSTÈME PERMETTANT DE DÉTERMINER DES RÉGIONS DE FIBRILLATION AURICULAIRE POUR ABLATION

(30) Priority: 14.01.2016 US 201662278676 P; 12.01.2017 US 201715404226
(43) Date of publication of application: 21.10.2020
(62) Divisional of application: 17151634.7
(73) Proprietor: Biosense Webster (Israel) Ltd., Yokneam 2066717 (IL)
(72) Inventor: URMAN, Roy, 2066717 Yokneam (IL); ZEIDAN, Ziyad, 2066717 Yokneam (IL); GOLDBERG, Stanislav, 2066717 Yokneam (IL); HAYAM, Gal, 2066717 Yokneam (IL); BAR-TAL, Meir, 2066717 Yokneam (IL); ZRIHEM, Yaniv Ben, 2066717 Yokneam (IL); VERMA, Atul, Toronto, Ontario M3B 1R1 (CA); AMOS, Yariv Avraham, 2010400 Tzorit (IL); HOUBEN, Richard P. M., 3620 Lanaken (BE)
(74) Representative: Carpmaels & Ransford LLP

(56) References cited:
- US-A1- 2011 230 775
- US-A1- 2014 005 563
- US-A1- 2014 081 114
- US-A1- 2015 216 438
- HOUBEN R P M ET AL: "S-wave predominance of epicardial electrograms during atrial fibrillation in humans: Indirect evidence for a role of the thin subepicardial layer", HEART RHYTHM, ELSEVIER, US, vol. 1, no. 6, 1 December 2004 (2004-12-01), pages 639 - 647, XP004680968, ISSN: 1547-5271, DOI: 10.1016/J.HRTHM.2004.08.015

## Description

### FIELD OF INVENTION

The present invention relates to systems for determining regions of interest to be ablated for treatment of cardiac arrhythmia, such as atrial fibrillation, and, more particularly, to systems for determining atrial fibrillation regions of interest to be ablated using maps representing detected electrical activity of the heart and maps representing spatio-temporal manifestations of the conditions indicative of the electrical activity of the heart.

### BACKGROUND

Cardiac arrhythmia includes different types of abnormal or irregular heart rhythms, such as, for example, atrial fibrillation (AF), which is characterized by rapid and irregular beating. Under normal heart conditions, a heartbeat is produced by electrical pulses (i.e., signals) which originate in the upper chambers (i.e., atria) of the heart and pass through the atria through the atrioventricular (AV) node to a pair of lower chambers (i.e., ventricles) of the heart. As the signals pass through the atria, the atria contract and pump blood from the atria into the ventricles. As the signals pass through the AV node to the ventricles, the ventricles are caused to contract, pumping out blood from the heart to the body. During conditions of AF, however, the signals in the atria become chaotic and cause the heart to beat irregularly.

AF can negatively affect the physical, psychological and emotional quality of a person's life. AF can progressively increase in severity and frequency and, if left untreated, may lead to chronic fatigue, congestive heart failure or stroke. One type of AF treatment includes prescribed medications, such as rhythm control medications and medications used to manage the increased risk of stroke. These medications must be taken daily and indefinitely. Another type of AF treatment includes cardioversion, which attempts to restore a normal heart rhythm by providing electric shocks to the heart through electrodes placed on the chest. In some persistent types of AF, cardioversion is either ineffective or cannot be attempted.

Recent approaches for treating AF include minimally invasive ablation procedures (e.g., catheter ablation) in which the heart tissue is ablated to terminate electrical pathways and block faulty electrical impulses that can cause heart rhythm disorders.
US2014005563A1 discloses a method for visualization of electrophysiology information that includes storing electroanatomic data in memory, the electroanatomic data representing electrical activity on an anatomic region within a patient's body over a time period. An interval within the time period is selected in response to a user selection. A visual representation of physiological information for the user selected interval can be generated by applying at least one analysis method to the electroanatomic data. The visual representation can spatially represented on a graphical representation of the anatomic region within the patient's body.
US2011230775A1 discloses imaging apparatus for imaging a heart. An imaging apparatus for imaging a heart is provided, wherein the imaging of the heart is improved such that conclusions about regions of the heart having an abnormal behaviour can be made more accurate and more optimal. The imaging apparatus comprises a first site determination unit for determining a first site of the heart comprising a first property type like a fractionated electrogram (70,71,74,75) and a second site determination unit for determining a second site comprising a second property type like a ganglionated plexus (72,73). The first site and the second site are causally related and displayed on a display unit. Since the displayed first and second sites are causally related to each other, a further information is given, i.e. the causal relation, which assists a user in finding regions of the heart showing an abnormal behaviour.
US2015216438A1 discloses integrated analysis of electrophysiological data. A method can include analyzing non-invasive electrical data for a region of interest (ROI) of a patient's anatomical structure to identify one or more zones within the ROI that contain at least one mechanism of distinct arrhythmogenic electrical activity. The method also includes analyzing invasive electrical data for a plurality of signals of interest at different spatial sites within each of the identified zones to determine intracardiac signal characteristics for the plurality of sites within each respective zone. The method also includes generating an output that integrates the at least one mechanism of distinct arrhythmogenic electrical activity for the one or more zones with intracardiac signal characteristics for the plurality of sites within each respective zone.

### SUMMARY

The present invention provides a system, a non-transitory computer readable medium and a computer program product as defined in the appended claims. A method of determining target heart ablation regions is provided which includes detecting, via a plurality of sensors, electro-cardiogram (ECG) signals. Each ECG signal is detected via one of the plurality of sensors and indicates electrical activity of a heart over time. The method also includes determining, for each of the plurality of ECG signals, a plurality of local activation times (LATs) occurring over time. Each LAT indicates a time of electrical activation for an area the heart. The method further includes generating, based on the determined plurality of LATs of each of the plurality of ECG signals, mapping information for one or more maps representing the electrical activity of the heart and determining a region of interest (ROI) of the heart by identifying the ROI as a region of the heart exhibiting conditions indicative of cardiac arrhythmia based on the generated mapping information for the one or more maps representing the electrical activity of the heart.

A system for determining target heart ablation regions is provided which includes a plurality of sensors each configured to detect one of a plurality of electro-cardiogram (ECG) signals each indicating electrical activity of a heart over time. The system also includes a processing device which includes one or more processors configured to determine, for each of the plurality of ECG signals, a plurality of local activation times (LATs) occurring over time. Each LAT indicates a time of electrical activation for an area the heart. The one or more processors are further configured to generate, based on the determined plurality of LATs of each of the plurality of ECG signals, mapping information for one or more maps representing the electrical activity of the heart and determine a region of interest (ROI) of the heart by identifying the ROI as a region of the heart exhibiting conditions indicative of cardiac arrhythmia based on the generated mapping information for the one or more maps representing the electrical activity of the heart.

A non-transitory computer readable medium is provided which includes instructions for causing a computer to execute a method of determining target heart ablation regions. The instructions include detecting, via a plurality of sensors, electro-cardiogram (ECG) signals. Each ECG signal is detected via one of the plurality of sensors and indicates electrical activity of the heart over time. The instructions also include determining, for each of the plurality of ECG signals, a plurality of local activation times (LATs) occurring over time. Each LAT indicates a time of electrical activation for an area the heart. The instructions further include generating, based on the determined plurality of LATs of each of the plurality of ECG signals, mapping information for one or more maps representing the electrical activity of the heart and determining a region of interest (ROI) of the heart by identifying the ROI as a region of the heart exhibiting conditions indicative of cardiac arrhythmia based on the generated mapping information for the one or more maps representing the electrical activity of the heart.

### BRIEF DESCRIPTION OF THE DRAWINGS

A more detailed understanding can be had from the following description, given by way of example in conjunction with the accompanying drawings wherein:
FIG. 1 is a block diagram illustrating an exemplary classification of AF used with embodiments disclosed herein;
FIG. 2 is a block diagram illustrating an exemplary system used to determine potential ablation ROIs for use with embodiments disclosed herein; and
FIGS. 3A and 3B are portions of a flow diagram illustrating an exemplary method of determining a potential ablation ROI for use with embodiments disclosed herein.

### DETAILED DESCRIPTION

Conventional methods and systems used for catheter ablation typically include inserting the catheter through an incision in the skin and guided up to the heart. Before ablation is performed, intra-cardiac electrocardiogram (IC ECG) signals of the heart are acquired via electrodes placed at different areas of the heart. The signals are monitored and used to provide information to determine whether one or more areas of the heart are causing the irregular heart rhythm. The conventional methods and systems used to determine these areas to be ablated, however, are time consuming (e.g., several hours) and rely on medical personnel with specific expertise and experience (typically requiring many hours of training).

Embodiments disclosed herein employ systems, apparatuses and methods of determining potential regions of interest (ROIs) to be targeted for ablation. Various mapping techniques are utilized to generate mapping information for one or more maps of the electro-physical conditions of the AF substrate and mapping information for one or more maps representing a spatio-temporal manifestation of the AF process to provide efficient and accurate determination of potential or target ablation ROIs. Mapping techniques utilize various parameters (e.g., cycle, earliness, R-S complex, conduction velocity (CV), block and fractionation) of acquired IC ECG signals and detected local activation times (LATs) to identify potential evidence of drivers and perpetuators of the AF substrate. Identification of the evidence of potential drivers and perpetuators is used to provide mapping (e.g., driver maps and perpetuator maps) of the AF substrate. Mapping techniques also include utilizing the various parameters of the acquired IC ECG signals and detected local activation times to generate mapping information for providing maps (e.g., activation/wave maps, CV maps, fractionation maps, voltage maps and block maps) which potentially represents the spatio-temporal manifestation of the AF process. The mapping of the spatio-temporal manifestation of the AF process can be used in addition to, or alternative to, the mapping of the AF substrate to identify potential ablation ROIs. The mapping techniques are used to potentially reduce AF map analysis training time, increase success rates resulting from ablation and facilitate efficient interpretation of AF maps. For simplification purposes, embodiments described herein refer to systems and methods used for the treatment of AF. It is noted however, embodiments may be used for the treatment of any type of cardiac arrhythmia including different types of abnormal or irregular heart rhythms.

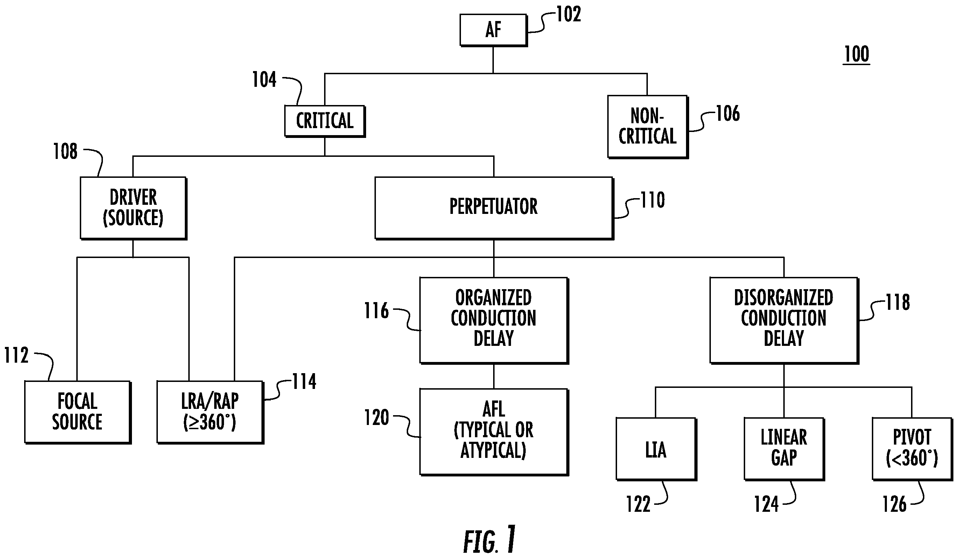

FIG. 1 is a block diagram illustrating an exemplary classification of AF used with embodiments disclosed herein. The exemplary classification in FIG. 1 distinguishes between critical and non-critical AF as well as between drivers and perpetuators of AF and their relative spatio-temporal patterns.

For example, as shown in FIG. 1, an irregular heart rhythm characterized as AF 102 is classified as critical 104 or non-critical 106. Examples of non-critical AF 106 include paroxysmal (i.e., intermittent) irregular heart rhythm episodes in which the heartbeat often normalizes as quickly as within a few seconds or after a few hours, and persistent irregular heart rhythm episodes in which a normal heart may be restored by rhythm medical therapy or a procedure (e.g., cardioversion). Examples of critical AF 104 include longstanding persistent irregular heart rhythm episodes that continue for longer periods of time (e.g., more than a year) in which the heart is in a constant state of AF and the condition is considered permanent.

Critical AF can be classified according to characteristics (e.g., areas of activation) that can be derived from IC ECG signals. Areas of activation may be identified as potential contributing factors to AF. As shown in FIG. 1, critical AF is classified according to different areas of activation, including a potential driver of AF (hereinafter "driver") or potential source of AF (hereinafter "source") 108 and a potential perpetuator 110 of AF (hereinafter "perpetuator"). A driver 108 is an area of activation (e.g., in the atria) where electrical pulses originate to stimulate the heart to contract and which can potentially contribute to AF, for example, by producing fibrillatory conduction to other areas of the atria. A perpetuator 110 is an area of sustained activation (e.g., electrophysiological process/substrate) which can also potentially contribute to AF.

Drivers 108 and perpetuators 110 may be represented (e.g., mapped) according to their spatio-temporal manifestation. As shown in FIG. 1, drivers 108 and perpetuators 110 are classified by exemplary spatio-temporal manifestation types, including focal sources (foci) 112 and localized rotational activation (LRA) sources or rotational activation patterns (RAPs) sources 114. A focal source is a type of driver originating at a small area of the atria which spreads centrifugally from a single point. A RAP 114 source is an irregular region of the heart where the electrical pulses rotate at least 360 degrees about a center area.

FIG. 1 also shows different types of perpetuators 110, including one type which exhibits organized conduction delay 116 and another which exhibits disorganized conduction delay 118. Another type of perpetuator 110 shown in FIG. 1 includes atrial flutter (AFL) 120, characterized by organized conduction delay 116 as well as localized irregular activation (LIA) 122, linear gaps 124 and pivots 126 (i.e., electrical pulses that rotate less than 360 degrees about a center area), characterized by disorganized conduction delay 118. Also, the RAP source 114 is shown as both a driver 108 and a perpetuator 110. Drivers 108 and perpetuators 110 are, for example, separately mapped to facilitate identification of driver types and/or perpetuator types and provide efficient and accurate determination of potential ablation ROIs.

Mapping and identification of drivers 108 and perpetuators 110 can also be based on one or more additional factors which may potentially contribute to AF or parameters which may potentially characterize the AF substrate (i.e., the AF process itself) and/or the manifestation of the AF process. For example, AF parameters or AF factors used to identify potential focal sources 108 include omnidirectional activation spread of activation from a point, earliness (e.g., focal source which starts after an excitable gap), triggers such as fast firing (e.g., short cycle-length and high dominant frequency) foci and breakthroughs (e.g., pulmonary veins (PV), free wall and transmural, endocardial and epicardial) and micro re-entry circuit which manifests as focal source and short-radius re-entry circuits which can manifest as a driver 108 depending on the specific anisotropic structure of the central obstacle.

AF parameters or AF factors used to map and identify RAP sources 114 include, for example, repetitive cycles, rotors which can manifest as a driver source 108, structural or functional anisotropy (e.g., localized or distributed), and short-radius re-entry circuits which can manifest as either a driver 108 or a perpetuator 110, depending on specific anisotropic structure of the central obstacle.

AF parameters or AF factors used to map and identify perpetuators 110 include, for example, extension (increased) path length, anatomical (pathological) block lines, fibrosis, stable functional block lines (e.g., areas of prolonged refractoriness), criticality (e.g., shortest path around block line > path length) and fibrillatory conduction factors (e.g., dissociated waves, re-entry circuit factors).

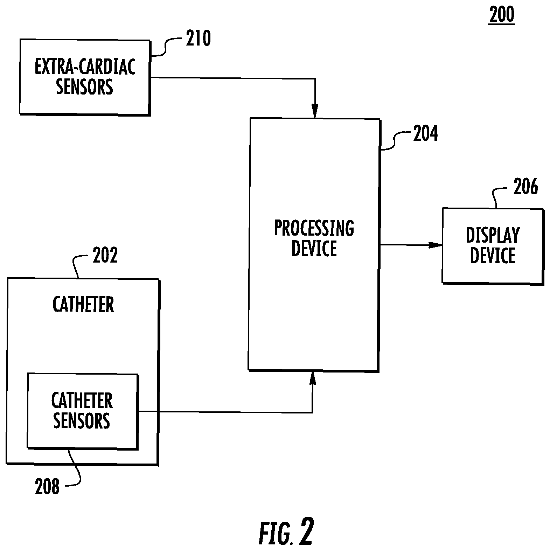

FIG. 2 is a block diagram illustrating an exemplary system 200 used to determine AF ROIs for ablation for use with embodiments disclosed herein. As shown in FIG. 2, the system 200 includes a catheter 202, a processing device 204 and a display device 206. Catheter 202 includes an array of catheter sensors (e.g., electrodes) each configured to detect electrical activity (electrical signals) of an area of the heart over time. When an IC ECG is performed, each electrode detects the electrical activity of an area of the heart in contact with the electrode. The system 200 also includes extra-cardiac sensors 210 (e.g., electrodes on the skin of a patient) configured to detect electrical activity of the heart via detection of electrical changes on the skin due to the electro-physiologic pattern of the heart.

The detected IC ECG signals and the detected extra-cardiac signals are processed (e.g., recorded over time, filtered, fractionated, mapped, combined, interpolated, etc.) by processing device 204 and displayed on display device 206.

Embodiments may include any number of sensors 210 used to detect ECG signals, including sensors used to detect IC ECG signals and extra-cardiac ECG signals. For simplification purposes, systems and methods described herein refer to the detection and use of IC ECG signals. It is noted, however, that embodiments may utilize IC ECG signals or extra-cardiac ECG signals or a combination of both IC ECG signals and extra-cardiac ECG signals.

Processing device 204 may include one or more processors each configured to process the ECG signals. Each processor of processing device 204 may be configured to record ECG signals over time, filter ECG signals, fractionate ECG signals into signal components (e.g., slopes, waves, complexes), map ECG signals, combine ECG signal information, map and interpolate mapping information, etc.

Display device 206 may include one or more displays each configured to display ECG signals, ECG signal information, maps of the AF process and maps representing a spatio-temporal manifestation of the AF process.

The catheter sensors 208 and the extra cardiac sensors 210 may be in wired or wireless communication with processing device 204. Display device 206 may also be in wired or wireless communication with processing device 204.

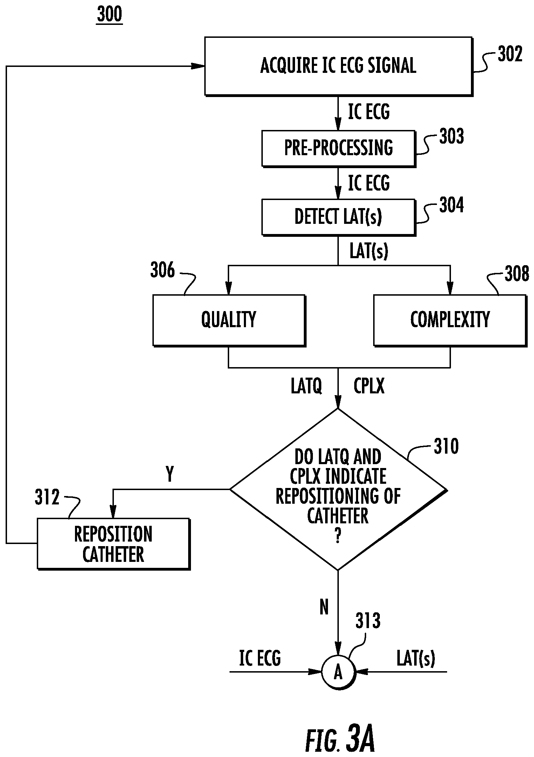

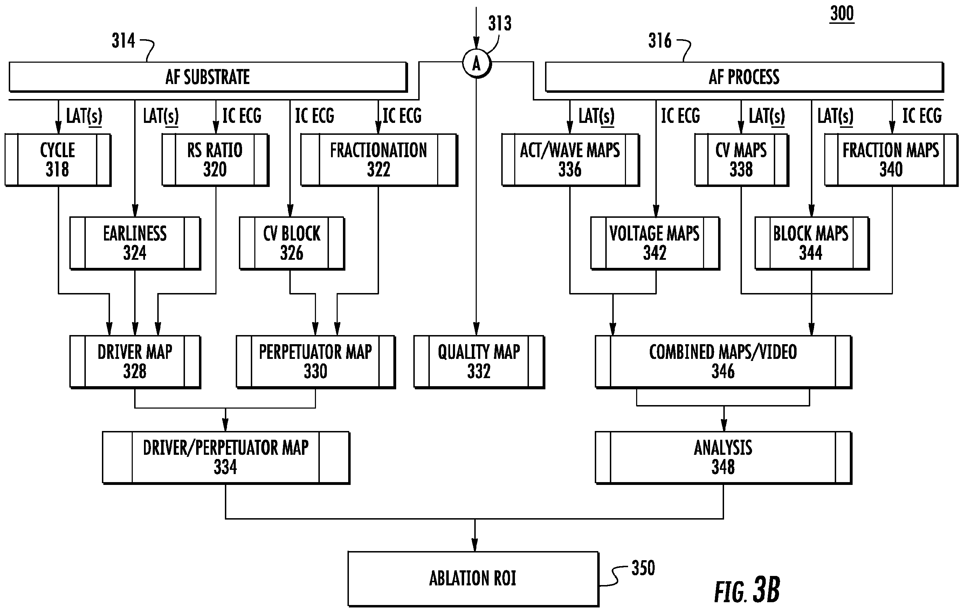

FIGS. 3A and 3B are portions of a flow diagram illustrating an exemplary method 300 of determining a potential ablation ROI. The method 300 employs a mapping taxonomy which includes, from its core moving outward, an IC ECG layer, a pre-processing layer, a LAT detection layer, a map segmentation layer, a map interpolation layer and a map interpretation layer.

FIG. 3A illustrates a portion of exemplary method 300. As shown in block 302 of FIG. 3A, the method 300 includes, as part of the IC ECG layer, acquiring an IC ECG signal which represents electrical activity of an area of the heart. The IC ECG signal acquired at block 302 is, for example, acquired from one of a number of electrodes in contact with different areas of the heart. After acquisition of the IC ECG (302), the method 300 includes, as part of the pre-processing layer, pre-processing of the acquired ECG signal, as shown in block 302 of FIG. 3A, The pre-processing may include execution of one or more algorithms, such as for example, cancellation of ventricular far field signals, baseline correction, and noise reduction. Ventricular far field detection may include, for example, a spatial averaging method (SAM), a temporal averaging method (TAM), a system identification method (SIM) and principal component analysis (PCA).

For each IC ECG signal acquired at block 302, one or more LATs of the corresponding pre-processed IC ECG signal is (are) detected at block 304. The LAT quality (shown as LATQ in FIG. 3A) of each signal is determined at block 306 as part of an exemplary LAT detection layer. The AF complexity (shown as CPLX in FIG. 3A) of the signal is determined at block 308.

As shown at decision point 310, the method 300 includes determining whether to reposition the catheter based on the LAT quality of the signal and the AF complexity. A typical characteristic of high quality IC ECGs is little base line wander (e.g., low baseline vs. IC ECG RMS amplitude, limited ventricular far-field potentials vs. IC ECG RMS amplitude). IC ECG signals characteristics include discernable atrial complexes (e.g., confined (~50ms) complexes separated by isoelectric segments repeating slopes, 50-200ms interval; about 150ms median) during AF. High quality complexes characteristic typically have considerable amplitudes and steep downward slopes (vs. upward slopes) within complexes. Characteristics of the IC ECG signals may be combined into a single measurable characteristic or parameter (e.g., having a measurable value of 0%-100%) to define LAT quality. The LAT quality may be compared to the AF complexity to determine whether to reposition the catheter.

In some embodiments, quality is defined by an ability to map AF for a level of AF complexity. Determining whether to reposition the catheter may include generating a map and determining whether the generated map can be used (e.g., is adequate) to map AF based on whether a level of coverage of a mapping electrode meets (e.g., matches) a level of AF complexity. The ability to map AF for a level of AF complexity may include meeting a map threshold level (e.g., adequate level, trustworthy level). A single parameter (i.e., mapping coverage) is used to define a level of coverage of the mapping electrode. Examples of characteristics that are combined to define the mapping coverage include: (1) contact of the mapping electrode (e.g., contact with active tissue (wall) related to covered area and LAT accuracy); (2) resolution of the electrodes (e.g., distances and electrode sensitivity radii between electrodes, including mean, minimum and maximum and distances); and (3) quality of the IC ECG and associated annotations provided by a detection algorithm.

AF complexity may include complexity of activation during AF creating wave dissociation (block lines), fusion and wave curvature. Accordingly, a map may be determined as a map which can be used (e.g., trustworthy or adequate) to map AF when, given a certain level of AF complexity (e.g., measured along y-axis), the mapping coverage (including signal and annotation quality measured along x-axis) is sufficient to map the AF complexity. If not, the trustworthiness of the map may become compromised or inadequate.

Signals may then be analyzed using the trustworthy or adequate maps to determine whether the catheter should be repositioned. If it is determined at decision point 310 to reposition the catheter, the catheter (e.g., catheter 202) is repositioned at block 312 and a new IC ECG signal is acquired at block 302. If it is determined at decision point 310 that the catheter should be repositioned, the method 300 continues to "point A" 313 (shown in FIG. 3A and FIG. 3B).

FIG. 3A illustrates the acquiring of a single IC ECG signal for simplification purposes. In practice, however, multiple signals are acquired for each of the plurality of electrodes contacting the heart. Each IC ECG signal acquired at block 202 and the one or more LATs detected for each signal at block 204 are received at "point A" 313.

Figure 3B illustrates exemplary methods which may be used to determine potential ablation ROIs. As shown FIG. 3B, each acquired IC ECG signal and the one or more detected LATs for each signal are used to generate maps of the AF process that includes the electro-physical conditions of the AF substrate (indicated as the AF Substrate 314 in FIG. 3B) and maps representing a spatio-temporal manifestation of the AF process (indicated as the AF Process 316 in FIG. 3B) as part of an exemplary map segmentation layer.

For example, with regard to the AF Substrate 314 shown in FIG. 3B, the one or more detected LATs are used to independently determine one or more factors or parameters which may contribute to AF. The left side of FIG. 3B illustrates methods which characterize the AF substrate by collecting information over a predefined window of time while assessing a mean interval (e.g., cycle) based on a difference of subsequent LATs 318, first activated (earliness) 324, and morphological aspects of the IC ECG including RS-ratio 320 and fractionation 322 (e.g., fractionated electrograms). For example, the detected LATs are used to independently determine cycle information (e.g., cycle lengths) at block 318 and earliness information (e.g., earliest activation times, early drivers which start after an excitable gap) at block 324. Each IC ECG signal is also used to independently determine R-S complex information (e.g., ratio of R wave to S wave) at block 320 and information obtained by fractionation (e.g., slope information, information indicating an incidence of source behavior presented as the earliest activation from one of a plurality of electrodes, such as showing a percentage that the associated electrode was activated earlier than neighbouring electrodes) of the IC ECG signals at block 322 and CV Block information (e.g., information indicating slowed or blocked conduction (i.e., progression) of electrical impulses through the heart, such as the conduction time (CT) for the electrical pulse to travel a distance in the heart, the path length (i.e., the distance) and the CV of the electrical pulse) at block 326.

As shown, a driver map 328 is generated from the cycle information 318, the earliness information 324 and the R-S complex information 320. A perpetuator map 330 is generated from the CV Block information 326 and the fractionation information 322. As shown, the information used to generate the driver map 328 and the information used to generate the perpetuator map 330 are combined (e.g., a single map, overlaid maps or adjacent maps in one display area) to generate a combined driver/perpetuator map 334. The combined driver/perpetuator map 334 may then be used (e.g., interpolated as part of an exemplary map interpolation layer) to determine one or more ablation ROIs 350.

With regard to the AF Process 316 shown in FIG. 3B, the one or more detected LATs are used to independently generate activation/wave maps 336, CV maps 338 (e.g., maps generated from the CT, the path length and/or the CV of the electrical pulse) and block maps 344 (e.g., maps generated from information indicating a block in the conduction of the signal).

Activation/wave maps may, for example, include a map representing an incidence of source behavior presenting the earliest activation of one of a plurality of electrodes restricted by the same wave, such as indicating a percentage of activation waves detected by a corresponding electrode activated earlier than neighboring electrodes though restricted by neighbors activated by the same wave. Activation Wave maps may, for example, also include a map representing the incidence of electrode positions associated with a fibrillation wave start.

Each IC ECG signal is used to independently generate voltage maps 342 and fraction maps 340. The information used to generate maps 336-344 is combined to provide combined maps or video 346. In some embodiments, the information used to generate the activation/wave maps 336 and Voltage maps 342 is combined to generate a combined activation/wave/voltage map or video and the information used to generate the CV maps 338, the block maps 344 and the fraction maps 340 are combined to generate a combined CV/block/fraction map or video. The combined maps/video 346 are analyzed (e.g., interpreted by medical personnel as part of an exemplary map interpretation layer) at block 348 to determine ROIs to be ablated at block 350. The combined maps/video 346 represent a spatio-temporal manifestation of the AF process which can be easily visualized and interpreted, facilitating an efficient and accurate process for determination of ROIs for ablation. Determined ROIs may be represented (e.g., displayed), for example, by color, by 3-D contour on a 4-D map, by icons (e.g., dynamically changing icons), etc.

In some embodiments, both the combined driver/perpetuator map 334 and the combined maps/video 346 are used to determine ROIs for ablation at block 350. In some embodiments either the combined driver/perpetuator map 334 or the combined maps/video 346 are used to determine ROIs for ablation at block 350. For example, the combined driver/perpetuator map 334 can be used to determine ROIs for ablation at block 350 without using (e.g., viewing, analyzing) the combined maps/video 346.

In some embodiments, the quality map 332 is also used in combination with the combined driver/perpetuator map 334 and/or the combined maps/video 346 to determine ROIs for ablation at block 350. The quality map 332 is used to determine the trustworthiness of the generated maps (e.g., driver map 328, perpetuator map 330 and driver/perpetuator map 334) related to AF substrate 314 and the generated maps (e.g., activation/wave maps 336, CV maps 338, fraction maps 340, voltage maps 342 and block maps 344) related to the AF process 316 parameters. If the quality of the quality map is low, the generated maps are less trusted and appointing an ablation ROI (350) must be regarded with an increase level of care (e.g., by a physician) compared to when the quality map indicates high quality signals (IC ECGs) as the basis for the generated maps.

In some embodiments, determining ROIs for ablation at block 350 includes appointing or selecting one or more ablation sites for use in determining one or more ROIs for ablation. For example, ablation sites may be appointed or selected from driver evidence and perpetuator evidence (e.g., determined from the driver map 328, the perpetuator map 330 or the combined driver/perpetuator map 334) and ROIs may be determined based on the appointed sites.

The maps and mapping techniques disclosed herein potentially: (i) reduce AF map analysis training time; (ii) reduce time to determine ROIs for ablation; (iii) facilitate efficient interpretation of AF maps; and (iv) increase ablation success rates for ablation aimed at isolation and extinguishing of drivers, path lengthening, slowing of re-entry circuits, fibrillatory conduction and fractionated potentials.

It should be understood that many variations are possible based on the disclosure herein. Although features and elements are described above in particular combinations, each feature or element can be used alone without the other features and elements or in various combinations with or without other features and elements.

The methods provided include implementation in a general purpose computer, a processor, or a processor core. Suitable processors include, by way of example, a general purpose processor, a special purpose processor, a conventional processor, a digital signal processor (DSP), a plurality of microprocessors, one or more microprocessors in association with a DSP core, a controller, a microcontroller, Application Specific Integrated Circuits (ASICs), Field Programmable Gate Arrays (FPGAs) circuits, any other type of integrated circuit (IC), and/or a state machine. Such processors can be manufactured by configuring a manufacturing process using the results of processed hardware description language (HDL) instructions and other intermediary data including netlists (such instructions capable of being stored on a computer readable media). The results of such processing can be maskworks that are then used in a semiconductor manufacturing process to manufacture a processor which implements methods described herein.

The methods or flow charts provided herein can be implemented in a computer program, software, or firmware incorporated in a non-transitory computer-readable storage medium for execution by a general purpose computer or a processor. Examples of non-transitory computer-readable storage mediums include a ROM, a random access memory (RAM), a register, cache memory, semiconductor memory devices, magnetic media such as internal hard disks and removable disks, magnetooptical media, and optical media such as CD-ROM disks, and digital versatile disks (DVDs).

## Claims

1. A system (200) for determining target heart ablation regions, the system comprising:
a plurality of sensors (208, 210) configured to detect a plurality of electro-cardiogram (ECG) signals each indicating electrical activity of a heart over time, each of the plurality of sensors configured to detect one of the ECG signals;
a processing device (204) comprising one or more processors configured to:
determine, for each of the plurality of ECG signals, a plurality of local activation times (LATs) occurring over time, each LAT indicating a time of electrical activation for an area the heart;
generate, based on the determined plurality of LATs of each of the plurality of ECG signals, mapping information for one or more maps representing the electrical activity of the heart; and
determine a region of interest (ROI) of the heart by identifying the ROI as a region of the heart exhibiting conditions indicative of cardiac arrhythmia based on the generated mapping information for the one or more maps representing the electrical activity of the heart,
wherein the ROI is identified as exhibiting conditions indicative of atrial fibrillation (AF),
wherein generated mapping information for the one or more generated maps representing the electrical activity of the heart comprises generating driver mapping information indicating potential AF drivers of AF, and
wherein the driver mapping information is generated according to time intervals between the LATs, information indicating earliness of the LATs and ratios of amplitudes of R-waves of the ECG signals and amplitudes of S-waves of the ECG signals.

2. The system of claim 1, wherein generated mapping information for the one or more generated maps representing the electrical activity of the heart further comprises generating perpetuator mapping information indicating potential AF perpetuators.

3. The system of claim 2, wherein the perpetuator mapping information is generated according to fractionations of the ECG signals and conduction velocities of the ECG signals.

4. The system of claim 1, wherein the processing device is further configured to:
generate, based on the determined plurality of LATs for each of the plurality of ECG signals, mapping information for one or more maps representing a spatio-temporal manifestation of the conditions indicative of cardiac arrhythmia;
determine the ROI of the heart based on the mapping information for the one or more maps representing the spatio-temporal manifestation of the conditions indicative of cardiac arrhythmia; and
provide, for display, the mapping information for the one or more maps representing the electrical activity of the heart and the mapping information for the one or more maps representing the spatio-temporal manifestation of the conditions indicative of cardiac arrhythmia.

5. The system of claim 4, wherein the processing device is further configured to generate the mapping information for the one or more generated maps representing the spatio-temporal manifestation of the conditions indicative of cardiac arrhythmia by generating:
activation wave mapping information, via the LATs indicating activation waves of the ECG signals;
conduction velocity mapping information, via the LATs, indicating conduction velocities of the ECG signals;
block mapping information, via the LATs, indicating areas of slowed or blocked conduction;
fractionation mapping information indicating fractionated portions of the ECG signals; and
voltage information indicating voltages of the ECG signals.

6. The system of claim 1, wherein the plurality of ECG signals comprise intra-cardiac electrocardiogram (IC ECG) signals of the heart acquired via a catheter (202), and
the processing device is further configured to determine whether to reposition the catheter based on an LAT quality of the IC ECG signals and a complexity of the IC ECG signals.

7. A non-transitory computer readable medium comprising instructions for causing a computer to:
determine, for each of a plurality of ECG signals, a plurality of local activation times (LATs) occurring over time, each LAT indicating a time of electrical activation for an area the heart;
generate, based on the determined plurality of LATs of each of the plurality of ECG signals, mapping information for one or more maps representing the electrical activity of the heart; and
determine a region of interest (ROI) of the heart by identifying the ROI as a region of the heart exhibiting conditions indicative of cardiac arrhythmia based on the generated mapping information for the one or more maps representing the electrical activity of the heart,
wherein the ROI is identified as exhibiting conditions indicative of atrial fibrillation (AF),
wherein generated mapping information for the one or more generated maps representing the electrical activity of the heart comprises generating driver mapping information indicating potential AF drivers of AF, and
wherein the driver mapping information is generated according to time intervals between the LATs, information indicating earliness of the LATs and ratios of amplitudes of R-waves of the ECG signals and amplitudes of S-waves of the ECG signals.

8. A computer program product comprising instructions for causing a computer to:
determine, for each of a plurality of ECG signals, a plurality of local activation times (LATs) occurring over time, each LAT indicating a time of electrical activation for an area the heart;
generate, based on the determined plurality of LATs of each of the plurality of ECG signals, mapping information for one or more maps representing the electrical activity of the heart; and
determine a region of interest (ROI) of the heart by identifying the ROI as a region of the heart exhibiting conditions indicative of cardiac arrhythmia based on the generated mapping information for the one or more maps representing the electrical activity of the heart,
wherein the ROI is identified as exhibiting conditions indicative of atrial fibrillation (AF),
wherein generated mapping information for the one or more generated maps representing the electrical activity of the heart comprises generating driver mapping information indicating potential AF drivers of AF, and
wherein the driver mapping information is generated according to time intervals between the LATs, information indicating earliness of the LATs and ratios of amplitudes of R-waves of the ECG signals and amplitudes of S-waves of the ECG signals.

## Patentansprüche

1. System (200) zum Bestimmen von Zielregionen für die Herzablation, wobei das System umfasst:
eine Vielzahl von Sensoren (208, 210), die konfiguriert sind, um eine Vielzahl von Elektrokardiogrammsignalen (EKG-Signalen) zu erkennen, die jeweils die elektrische Aktivität eines Herzens über die Zeit angeben, wobei jeder der Vielzahl von Sensoren konfiguriert ist, um eines der EKG-Signale zu erkennen;
eine Verarbeitungsvorrichtung (204), umfassend einen oder mehrere Prozessoren, konfiguriert zum:
Bestimmen, für jedes der Vielzahl von EKG-Signalen, einer Vielzahl von im Laufe der Zeit auftretenden lokalen Aktivierungszeiten (LATs), wobei jede LAT eine Zeit der elektrischen Aktivierung für einen Bereich des Herzens angibt;
Erzeugen, basierend auf der Vielzahl von LATs jeder der Vielzahl von EKG-Signalen, von Abbildungsinformationen für eine oder mehrere Abbildungen, die die elektrische Aktivität des Herzens darstellen; und
Bestimmen einer Region von Interesse (ROI) des Herzens durch Identifizieren der ROI als eine Region des Herzens, die Zustände aufzeigt, die auf Herzrhythmusstörungen hinweisen, basierend auf den erzeugten Abbildungsinformationen für die eine oder mehrere Abbildungen, die die elektrische Aktivität des Herzens darstellen,
wobei die ROI als Zustände aufzeigend identifiziert wird, die auf Vorhofflimmern (AF) hinweisen,
wobei die erzeugten Abbildungsinformationen für die eine oder mehreren erzeugten Abbildungen, die die elektrische Aktivität des Herzens darstellen, das Erzeugen von Treiberabbildungsinformationen umfassen, die auf potenzielle AF-Treiber von AF hinweisen und
wobei die Treiberabbildungsinformationen entsprechend den Zeitintervallen zwischen den LATs, Informationen, die die Frühzeitigkeit der LATs angeben, und Verhältnissen der Amplituden der R-Wellen der EKG-Signale und der Amplituden der S-Wellen der EKG-Signale erzeugt werden.

2. System nach Anspruch 1, wobei die erzeugten Abbildungsinformationen für die eine oder mehreren erzeugten Abbildungen, die die elektrische Aktivität des Herzens darstellen, ferner das Erzeugen von Fortsetzer-Abbildungsinformationen umfassen, die auf potenzielle AF-Fortsetzer von AF hinweisen.

3. System nach Anspruch 2, wobei die Fortsetzer-Abbildungsinformationen entsprechend den Fraktionierungen der EKG-Signale und den Leitungsgeschwindigkeiten der EKG-Signale erzeugt werden.

4. System nach Anspruch 1, wobei die Verarbeitungsvorrichtung ferner konfiguriert ist zum:
Erzeugen, basierend auf der Vielzahl von LATs für jede der Vielzahl von EKG-Signalen, von Abbildungsinformationen für eine oder mehrere Abbildungen, die eine räumlich-zeitliche Manifestation der Zustände darstellen, die auf Herzrhythmusstörungen hinweisen;
Bestimmen der ROI des Herzens basierend auf den Abbildungsinformationen für die eine oder mehreren Abbildungen, die die räumlich-zeitliche Manifestation der auf Herzrhythmusstörungen hinweisenden Zustände darstellen; und
Bereitstellung, zur Anzeige, der Abbildungsinformationen für die eine oder mehreren Abbildungen, die die elektrische Aktivität des Herzens darstellen, und der Abbildungsinformationen für die eine oder mehreren Abbildungen, die die räumlich-zeitliche Manifestation der auf Herzrhythmusstörungen hinweisenden Zustände darstellen.

5. System nach Anspruch 4, wobei die Verarbeitungsvorrichtung ferner konfiguriert ist zum Erzeugen von Abbildungsinformationen für die eine oder mehreren erzeugten Abbildungen, die die räumlich-zeitliche Manifestation der auf Herzrhythmusstörungen hinweisenden Zustände darstellen, durch Erzeugen:
von Aktivierungswellen-Abbildungsinformationen über die LATs, die die Aktivierungswellen der EKG-Signale angeben;
von Leitungsgeschwindigkeits-Abbildungsinformationen über die LATs, die die Leitungsgeschwindigkeiten der EKG-Signale angeben;
von Blockabbildungsinformationen über die LATs, die Bereiche verlangsamter oder blockierter Leitung angeben;
von Fraktionierungsabbildungsinformationen, die fraktionierte Teile der EKG-Signale angeben; und
von Spannungsinformationen, die die Spannungen der EKG-Signale angeben.

6. System nach Anspruch 1, wobei die Vielzahl von EKG-Signalen intrakardiale Elektrokardiogramm-Signale (IC-EKG-Signale) des Herzens umfasst, die über einen Katheter (202) erfasst werden, und
die Verarbeitungsvorrichtung ferner konfiguriert ist, zu bestimmen, ob der Katheter neu positioniert werden soll, basierend auf einer LAT-Qualität der IC-EKG-Signale und einer Komplexität der IC-EKG-Signale.

7. Nicht-transitorisches, computerlesbares Medium, umfassend Anweisungen, die einen Computer veranlassen zum:
Bestimmen, für jedes einer Vielzahl von EKG-Signalen, einer Vielzahl von im Laufe der Zeit auftretenden lokalen Aktivierungszeiten (LATs), wobei jede LAT eine Zeit der elektrischen Aktivierung für einen Bereich des Herzens angibt;
Erzeugen, basierend auf der Vielzahl von LATs jeder der Vielzahl von EKG-Signalen, von Abbildungsinformationen für eine oder mehrere Abbildungen, die die elektrische Aktivität des Herzens darstellen; und
Bestimmen einer Region von Interesse (ROI) des Herzens durch Identifizieren der ROI als eine Region des Herzens, die Zustände aufzeigt, die auf Herzrhythmusstörungen hinweisen, basierend auf den erzeugten Abbildungsinformationen für die eine oder mehrere Abbildungen, die die elektrische Aktivität des Herzens darstellen,
wobei die ROI als Zustände aufzeigend identifiziert wird, die auf Vorhofflimmern (AF) hinweisen,
wobei die erzeugten Abbildungsinformationen für die eine oder mehreren erzeugten Abbildungen, die die elektrische Aktivität des Herzens darstellen, das Erzeugen von Treiberabbildungsinformationen umfassen, die auf potenzielle AF-Treiber von AF hinweisen und
wobei die Treiberabbildungsinformationen entsprechend den Zeitintervallen zwischen den LATs, Informationen, die die Frühzeitigkeit der LATs angeben, und Verhältnissen der Amplituden der R-Wellen der EKG-Signale und der Amplituden der S-Wellen der EKG-Signale erzeugt werden.

8. Computerprogrammprodukt, umfassend Anweisungen, die einen Computer veranlassen zum:
Bestimmen, für jedes einer Vielzahl von EKG-Signalen, einer Vielzahl von im Laufe der Zeit auftretenden lokalen Aktivierungszeiten (LATs), wobei jede LAT eine Zeit der elektrischen Aktivierung für einen Bereich des Herzens angibt;
Erzeugen, basierend auf der Vielzahl von LATs jeder der Vielzahl von EKG-Signalen, von Abbildungsinformationen für eine oder mehrere Abbildungen, die die elektrische Aktivität des Herzens darstellen; und
Bestimmen einer Region von Interesse (ROI) des Herzens durch Identifizieren der ROI als eine Region des Herzens, die Zustände aufzeigt, die auf Herzrhythmusstörungen hinweisen, basierend auf den erzeugten Abbildungsinformationen für die eine oder mehrere Abbildungen, die die elektrische Aktivität des Herzens darstellen,
wobei die ROI als Zustände aufzeigend identifiziert wird, die auf Vorhofflimmern (AF) hinweisen,
wobei die erzeugten Abbildungsinformationen für die eine oder mehreren erzeugten Abbildungen, die die elektrische Aktivität des Herzens darstellen, das Erzeugen von Treiberabbildungsinformationen umfassen, die auf potenzielle AF-Treiber von AF hinweisen und
wobei die Treiberabbildungsinformationen entsprechend den Zeitintervallen zwischen den LATs, Informationen, die die Frühzeitigkeit der LATs angeben, und Verhältnissen der Amplituden der R-Wellen der EKG-Signale und der Amplituden der S-Wellen der EKG-Signale erzeugt werden.

## Revendications

1. Système (200) permettant de déterminer des régions cibles d'ablation cardiaque, le système comprenant :
une pluralité de capteurs (208, 210) configurés pour détecter une pluralité de signaux d'électrocardiogramme (ECG) indiquant chacun une activité électrique d'un coeur au fil du temps, chacun parmi la pluralité de capteurs étant configuré pour détecter l'un des signaux ECG ;
un dispositif de traitement (204) comprenant un ou plusieurs processeurs configurés pour :
déterminer, pour chacun parmi la pluralité de signaux ECG, une pluralité de moments d'activation locale (LAT) se produisant au fil du temps, chaque LAT indiquant un moment d'activation électrique pour une zone du coeur ;
générer, en fonction de la pluralité déterminée de LAT de chacun parmi la pluralité de signaux ECG, des informations de cartographie pour une ou plusieurs cartes représentant l'activité électrique du coeur ; et
déterminer une région d'intérêt (ROI) du cœur en identifiant la ROI en tant que région du coeur présentant des conditions indiquant une arythmie cardiaque en fonction des informations de cartographie générées pour la ou les cartes représentant l'activité électrique du coeur,
dans lequel la ROI est identifiée comme présentant des conditions indiquant une fibrillation auriculaire (FA),
dans lequel les informations de cartographie générées pour la ou les cartes générées représentant l'activité électrique du coeur comprennent la génération d'informations de cartographie de facteur indiquant des facteurs potentiels de FA de la FA, et
dans lequel les informations de cartographie de facteur sont générées selon des intervalles de temps entre les LAT, des informations indiquant la précocité des LAT et des rapports d'amplitudes d'ondes R des signaux ECG et d'amplitudes d'ondes S des signaux ECG.

2. Système selon la revendication 1, dans lequel les informations de cartographie générées pour la ou les cartes générées représentant l'activité électrique du coeur comprennent en outre la génération d'informations de cartographie de continuateur indiquant des continuateurs potentiels de FA.

3. Système selon la revendication 2, dans lequel les informations de cartographie de continuateur sont générées selon des fractionnements des signaux ECG et des vitesses de conduction des signaux ECG.

4. Système selon la revendication 1, dans lequel le dispositif de traitement est configuré en outre pour :
générer, en fonction de la pluralité déterminée de LAT pour chacun parmi la pluralité de signaux ECG, des informations de cartographie pour une ou plusieurs cartes représentant une manifestation spatio-temporelle des conditions indiquant une arythmie cardiaque ;
déterminer la ROI du coeur en fonction des informations de cartographie pour la ou les cartes représentant la manifestation spatio-temporelle des conditions indiquant une arythmie cardiaque ; et
fournir, pour affichage, les informations de cartographie pour la ou les cartes représentant l'activité électrique du coeur et les informations de cartographie pour la ou les cartes représentant la manifestation spatio-temporelle des conditions indiquant une arythmie cardiaque.

5. Système selon la revendication 4, dans lequel le dispositif de traitement est configuré en outre pour générer les informations de cartographie pour la ou les cartes générées représentant la manifestation spatio-temporelle des conditions indiquant une arythmie cardiaque en générant :
des informations de cartographie d'onde d'activation, par l'intermédiaire des LAT indiquant des ondes d'activation des signaux ECG ;
des informations de cartographie de vitesse de conduction, par l'intermédiaire des LAT, indiquant des vitesses de conduction des signaux ECG ;
des informations de cartographie de blocage, par l'intermédiaire des LAT, indiquant des zones de conduction ralentie ou bloquée ;
des informations de cartographie de fractionnement indiquant des parties fractionnées des signaux ECG ; et
des informations de tension indiquant des tensions des signaux ECG.

6. Système selon la revendication 1, dans lequel la pluralité de signaux ECG comprennent des signaux d'électrocardiogramme intra-cardiaque (IC ECG) du cœur acquis par l'intermédiaire d'un cathéter (202), et
le dispositif de traitement est configuré en outre pour déterminer s'il faut repositionner le cathéter en fonction d'une qualité de LAT des signaux IC ECG et d'une complexité des signaux IC ECG.

7. Support non transitoire lisible par ordinateur comprenant des instructions permettant d'amener un ordinateur à :
déterminer, pour chacun parmi une pluralité de signaux ECG, une pluralité de moments d'activation locale (LAT) se produisant au fil du temps, chaque LAT indiquant un moment d'activation électrique pour une zone du coeur ;
générer, en fonction de la pluralité déterminée de LAT de chacun parmi la pluralité de signaux ECG, des informations de cartographie pour une ou plusieurs cartes représentant l'activité électrique du coeur ; et
déterminer une région d'intérêt (ROI) du cœur en identifiant la ROI en tant que région du cœur présentant des conditions indiquant une arythmie cardiaque en fonction des informations de cartographie générées pour la ou les cartes représentant l'activité électrique du coeur,
dans lequel la ROI est identifiée comme présentant des conditions indiquant une fibrillation auriculaire (FA),
dans lequel les informations de cartographie générées pour la ou les cartes générées représentant l'activité électrique du cœur comprennent la génération d'informations de cartographie de facteur indiquant des facteurs potentiels de FA de la FA, et
dans lequel les informations de cartographie de facteur sont générées selon des intervalles de temps entre les LAT, des informations indiquant la précocité des LAT et des rapports d'amplitudes d'ondes R des signaux ECG et d'amplitudes d'ondes S des signaux ECG.

8. Produit-programme informatique comprenant des instructions permettant d'amener un ordinateur à :
déterminer, pour chacun parmi une pluralité de signaux ECG, une pluralité de moments d'activation locale (LAT) se produisant au fil du temps, chaque LAT indiquant un moment d'activation électrique pour une zone du coeur ;
générer, en fonction de la pluralité déterminée de LAT de chacun parmi la pluralité de signaux ECG, des informations de cartographie pour une ou plusieurs cartes représentant l'activité électrique du coeur ; et
déterminer une région d'intérêt (ROI) du cœur en identifiant la ROI en tant que région du cœur présentant des conditions indiquant une arythmie cardiaque en fonction des informations de cartographie générées pour la ou les cartes représentant l'activité électrique du coeur,
dans lequel la ROI est identifiée comme présentant des conditions indiquant une fibrillation auriculaire (FA),
dans lequel les informations de cartographie générées pour la ou les cartes générées représentant l'activité électrique du cœur comprennent la génération d'informations de cartographie de facteur indiquant des facteurs potentiels de FA de la FA, et
dans lequel les informations de cartographie de facteur sont générées selon des intervalles de temps entre les LAT, des informations indiquant la précocité des LAT et des rapports d'amplitudes d'ondes R des signaux ECG et d'amplitudes d'ondes S des signaux ECG.
